# EUROPÄISCHE PATENTSCHRIFT

(11) **EP 1 010 456 B1**
(45) Veröffentlichungstag und Bekanntmachung des Hinweises auf die Patenterteilung: **25.05.2005**
(21) Anmeldenummer: 99123029.3
(22) Anmeldetag: 19.11.1999
(51) Int. Cl.: B01D 63/02, A61M 1/18, B01D 65/00

(54) **Filtervorrichtung**
Filtering device
Appareil de filtration

(30) Priorität: 15.12.1998 DE 19857850
(43) Veröffentlichungstag der Anmeldung: 21.06.2000
(73) Patentinhaber: Fresenius Medical Care Deutschland GmbH, 61352 Bad Homburg v.d.H. (DE)
(72) Erfinder: Heilmann, Klaus, 66606 St. Wendel (DE); Breith, Gerhard, 66953 Pirmasens (DE); Raiko, Igor, 66606 St. Wendel (DE); Sander, Roland ., 66606 St. Wendel (DE); Fritzsche, Steffen Dr., 66636 Tholey (DE)
(74) Vertreter: Laufhütte, Dieter, Dr.-Ing.

(56) Entgegenhaltungen:
- DE-A- 2 836 007
- DE-A- 3 435 883
- DE-A- 3 711 695
- DE-A- 19 744 336
- US-A- 5 084 244

## Beschreibung

Die vorliegende Erfindung betrifft eine Filtervorrichtung zum Stoffaustausch zwischen zwei durch eine Membran getrennten Medien sowie eine Endkappe für eine derartige Filtervorrichtung.

Derartige Filtervorrichtungen werden üblicherweise als Dialysatoren, Hämofilter oder Ultrafilter verwendet. Die Filtervorrichtungen bestehen im allgemeinen aus einem rohrabschnittförmigen Gehäuse, das in seinen Endbereichen mit Endkappen versehen ist. In dem Gehäuse ist üblicherweise ein als Membran dienendes Rohrfaserbündel derart angeordnet, daß eine Abdichtung zwischen dem durch die Faserhohlräume gebildeten ersten Strömungsraum und einem zweiten Strömungsraum, der die Außenseite der Membran umgibt, zuverlässig gegeben ist.

Bei der Gestaltung der Ein- und/oder Ausströmkammer, die mit dem ersten Strömungsraum, d.h. mit dem Hohlfaserbündel, in Verbindung steht, besteht ein Problem darin, die Flüssigkeit dergestalt zu verteilen, daß die einzelnen Fasern des Hohlfaserbündels in etwa gleichmäßig mit Flüssigkeit beaufschlagt werden und bei der Verteilung der Flüssigkeit Totzonen vermieden werden.

Es sind Dialysatoren bekannt, bei denen der Zulauf der mit dem Hohlfaserbündel in Verbindung stehenden Kammer axial angeordnet ist, wobei die Achse des Strömungskanals in etwa durch den Mittelpunkt des Hohlfaserbündels verläuft. Hierbei ergibt sich im allgemeinen der Nachteil, daß die Fasern stark unterschiedlich genutzt werden und gleichzeitig Bereiche auftreten, in denen die Strömungsgeschwindigkeit in etwa bei Null liegt (Totzonen). Derartige Totzonen sind nach Abschluß einer Dialysebehandlung die Bereiche, in denen Patientenblut zurückbleibt. Zusätzlich besteht ein großer Unterschied zwischen der Strömungsgeschwindigkeit im Zulauf und in den Fasern. Durch den dadurch verursachten Geschwindiokeitsgradienten wird das Blut einer Streßsituation ausgesetzt.

Aus der DE-OS 26 46 358 ist eine Filtervorrichtung bekannt, bei der die Endkappen einen tangentialen Zulauf aufweisen und das Blut in einem Kanal auf einer Kreisbahn über die Enden der Hohlfasern geführt wird. Das Blut strömt tangential über die Hohlfaserenden. Um eine möglichst gleichmäßige Flüssigkeitsverteilung zu erreichen, ist vorgesehen, daß nur die auf der überströmten Kreisbahn befindlichen Bereiche der Vergußmasse mit Hohlfasern versehen sind, während der restliche Kernbereich keine Fasern aufweist. Dadurch wird einerseits eine gleichmäßige Belastung der Fasern erreicht, andererseits ergibt sich aufgrund des Fehlens von Hohlfasern im Zentrum des Gehäuses insgesamt jedoch eine verhältnismäßig geringe Kapazität bzw. nicht optimale Ausnutzung der Filtervorrichtung. Um eine einheitliche Kreisflußgeschwindigkeit des Blutes im Kanal zu erzielen, weist dieser in einer Ausführungsform eine in Strömungsrichtung abnehmende Querschnittsfläche auf.

Es ist auch vorgeschlagen worden, den Zulauf parallel zur Ebene der Faserenden zu legen und im Endbereich des Zulaufs einen Richtungswechsel des Mediums um 90° vorzusehen. Bedingt durch den geringen verfügbaren Raum und Beschränkungen einer Fertigung durch Spritzgußverfahren ist im allgemeinen nur ein abrupter Richtungswechsel möglich, was zu erheblichen Belastungen des Blutes führen würde.

Es ist die Aufgabe der vorliegenden Erfindung eine gattungsgemäße Filtervorrichtung dahingehend weiterzubilden, daß der Übergang des Blutes vom Zulauf in die Fasern belastungsarm erfolgt und die Fasern gleichmäßig beaufschlagt werden.

Diese Aufgabe wird erfindungsgemäß durch eine Filtervorrichtung gemäß Anspruch 1 gelöst. Daraus ergibt sich zum einen der Vorteil, daß die Blutströmung nur verhältnismäßig sanfte bzw. belastungsarme Änderungen der Geschwindigkeit in Richtung und Betrag erfährt. Die Strömungsgeschwindigkeit nähert sich bei der erfindungsgemäßen Vorrichtung beim Weg durch den im wesentlichen kreis- oder teilkreisförmigen Kanal langsam an den Wert der Geschwindigkeit in den Fasern an.

Zum anderen wird der Vorteil erreicht, daß das Blut auf dem Weg durch den Kanal durch die sich ändernde Querschnittsfläche annähernd radialsymmetrisch verteilt wird. Dies bedeutet, daß in jedem Winkelbereich in etwa der gleiche Betrag der Blutströmung den Kanal verläßt und radial nach außen fließt. Hieraus ergibt sich eine gleichmäßige Ausnutzung der einzelnen Fasern des Hohlfaserbündels und damit eine optimale Nutzung der Faserkapazität.

Im Gegensatz zu vorbekannten Lösungen kommt es bei der erfindungsgemäßen Anordnung zu keiner ausgeprägten Wirbelbildung, sondern zu einer vorwiegend gleichmäßigen radialsymmetrischen Verteilung der Flüssigkeit bzw. des Blutes.

Besonders vorteilhaft ist es, wenn die Hohlfasem in ihren Endbereichen in einer Vergußmasse aufgenommen sind, die sich parallel zu dem Kanal erstreckt. Die Vergußmasse ist üblicherweise scheibenförmig ausgeführt und die Hohlfaserenden sind in einer gleichmäßigen Verteilung in der Vergußmasse angeordnet.

Gemäß einer bevorzugten Ausgestaltung der vorliegenden Erfindung nimmt die Höhe und/oder Breite des Kanals in Strömungsrichtung ab. Besonders vorteilhaft ist es, wenn die Querschnittsfläche des Kanals linear mit dem Strömungsweg abnimmt. Durch eine derartige Ausgestaltung wird erreicht, daß pro Winkelbereich jeweils identische Mengen an Flüssigkeit bzw. Blut aus dem Kanal abgegeben werden, so daß eine annähernd radialsymmetrische Verteilung vorliegt.

Besonders vorteilhaft ist es, wenn ein sich radial erstreckender Zu- oder Ablauf vorgesehen ist, der in seinem einen Endbereich in den erfindungsgemäßen Kanal übergeht und in seinem anderen Endbereich die Mündung eines Anschlusses der Filtervorrichtung bildet.

Der Durchmesser des kreis- oder teilkreisförmigen Kanals kann vorteilhaft das 2- bis 4-fache, vorzugsweise das 2,5- bis 3,5-fache des Durchmessers des Zu- oder Ablaufes betragen.

In weiterer Ausgestaltung der vorliegenden Erfindung ist vorgesehen, daß der Durchmesser des kreis- oder teilkreisförmigen Kanals kleiner als 9/16, vorzugsweise kleiner als 3/8 des Durchmessers des Hohlfaserbündels ausgeführt ist.

Besonders vorteilhaft ist es, wenn der Kanal kreisförmig, d.h. umlaufend ausgeführt ist. Es ist somit nicht nur eine teilkreisförmige Ausführung des Kanals denkbar, sondern auch ein vollständiger Umlauf.

In weiterer Ausgestaltung der vorliegenden Erfindung ist vorgesehen, daß in den dem im wesentlichen kreis- oder teilkreisförmigen Kanal benachbarten Bereichen Führungselemente vorgesehen sind, mittels derer die Strömungsrichtung des den Kanal verlassenden Fluids beeinflußbar ist.

Die Führungselemente können als gekrümmte Rippen ausgeführt sein. Hierdurch wird es möglich, das Fluid, insbesondere das Blut, gleichmäßig und in definierter Art und Weise zu verteilen. Hierbei kann es zu der Ausbildung eines kleinen zentralen Wirbels kommen, der jedoch stets radialsymmetrisch ist.

Besonders vorteilhaft ist es, wenn die Rippen in Umfangsrichtung des Kanals äquidistant angeordnet sind. Darüber hinaus ist auch jede beliebige andere Anordnung von Rippen bzw. Führungselementen denkbar.

Das Hohlfaserbündel kann in einem rohrabschnittförmigen Gehäuse aufgenommen sein, wobei ein zweiter Strömungsraum durch den das Hohlfaserbündel umgebenden Gehäuseinnenraum gebildet wird und wobei die Ein- oder Ausströmkammer in einer mit dem Gehäuse stirnseitig in Verbindung stehenden Endkappe angeordnet ist. Es können in beiden Endbereichen des Gehäuses Endkappen vorgesehen sein.

Gemäß einer bevorzugten Ausführungsform der vorliegenden Erfindung erstreckt sich das Hohlfaserbündel über die gesamte Querschnittsfläche des Gehäuses. Ebenso ist es denkbar, daß nur Teilbereiche der Querschnittsfläche des Gehäuses bzw. der Vergußmasse mit Hohlfasem versehen sind.

Besonders vorteilhaft ist es, wenn jede der Endkappen zwei Anschlüsse aufweist, von denen einer mit dem ersten und einer mit dem zweiten Strömungsraum in Verbindung steht. Somit werden für jeden der Strömungsräume ein Zugang sowie ein Ablauf geschaffen, was entsprechend eine kontinuierliche Durchströmung beider Strömungsräume ermöglicht. Die Zuordnung als Zu- oder Ablauf ist nicht festgelegt, da die Anschlüsse je nach Bedarf vertauscht werden können.

In weiterer Ausgestaltung der vorliegenden Erfindung ist vorgesehen, daß die Anschlüsse radial angeordnet sind. Dies gilt entsprechend sowohl für die Blutseite, als auch für den Anschluß für die Dialyselösung, die üblicherweise durch den zweiten Strömungsraum geführt wird. Die radiale Anordnung der Anschlüsse kann zum einen strömungstechnische Vorteile haben. Zum anderen kann die Anordnung der Anschlüsse auch durch das zu verwendende Schlauchsystem bzw. durch die Halterung der Filtervorrichtung vorgegeben sein.

Gemäß einer bevorzugten Ausführungsform der vorliegenden Erfindung verlaufen die Mittellinien der Anschlüsse zueinander parallel. Es ist auch möglich, daß die Mittellinien nicht den Mittelpunkt der Endkappe durchlaufen.

Die Mündungen der Anschlüsse können in einer Ebene oder auch in zueinander parallelen Ebenen liegen. Die entsprechende Ausgestaltung ist im wesentlichen von der die Filtervorrichtung aufnehmenden Halterung abhängig, durch die ein rascher und zuverlässiger Wechsel der Filtervorrichtungen ermöglicht werden muß. In weiterer Ausgestaltung der vorliegenden Erfindung ist vorgesehen, daß die Anschlüsse in entgegengesetzte Richtungen weisen.

Besonders vorteilhaft ist es, wenn das Gehäuse sowie die Endkappen als Spritzgußteile ausgeführt sind. Hierdurch läßt sich eine verhältnismäßig einfache Herstellung gewährleisten, wobei zahlreiche unterschiedliche Ausführungsformen denkbar sind.

Die vorliegende Erfindung betrifft ferner eine Endkappe gemäß Anspruch 21.

Vorteilhafte Ausgestaltungen der Endkappe werden durch die Ausführungsformen gemäß der Ansprüche 1-20 wiedergegeben.

Weitere Einzelheiten und Vorteile der vorliegenden Erfindung werden anhand eines in der Zeichnung dargestellten Ausführungsbeispiels näher erläutert.

Es zeigen:
- Fig. 1:: eine Längsschnittansicht einer Endkappe mit Ein- oder Ausströmkammer mit teilkreisförmigem Kanal,
- Fig. 2:: eine teilweise Seitenansicht der Endkappe gemäß Fig. 1 gemäß Linie A-A,
- Fig. 3:: eine teilweise Seitenansicht der Endkappe gemäß Fig. 1 gemäß Linie B-B,
- Fig. 4:: eine Draufsicht auf die Endkappe gemäß Fig. 1,
- Fig. 5:: eine Detailansicht einer Schnittdarstellung gemäß Linie C-C in Fig. 4,
- Fig. 6:: eine Ansicht der Endkappe gemäß Fig. 1 von unten,
- Fig. 7:: eine schematische Darstellung der Größenverhältnisse von Zuoder Ablauf, Kanal und Hohlfaserbündel
- Fig. 8:: eine Längsschnittansicht einer Endkappe mit Ein- oder Ausströmkammer mit kreisförmigen, d.h. umlaufenden Kanal,
- Fig. 9:: eine Draufsicht auf die Endkappe gemäß Fig. 8,
- Fig. 10:: eine Detailansicht einer Schnittdarstellung gemäß der Linie D-D in Fig. 9,
- Fig. 11:: eine Ansicht der Endkappe gemäß Fig. 8 von unten und
- Fig. 12:: eine Detailansicht des kreisförmigen Kanals sowie der benachbar ten als Rippen ausgeführten Führungselemente gemäß Fig. 11

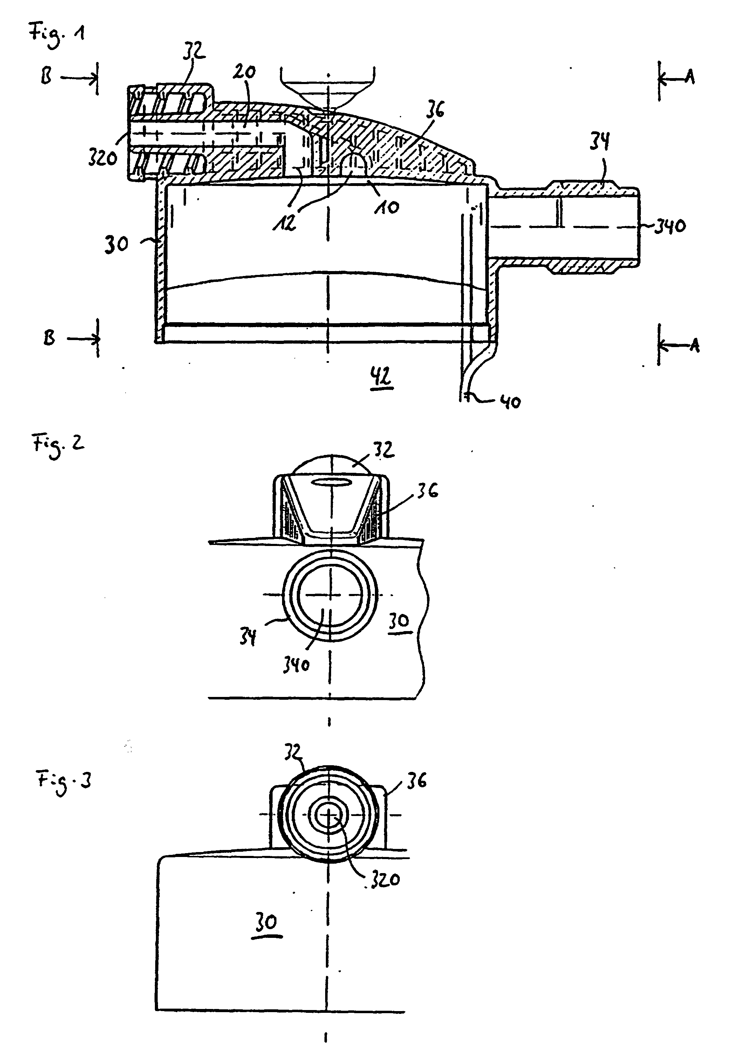

Fig. 1 zeigt in einer Längsschnittdarstellung die Endkappe 30, die flüssigkeitsdicht mit dem Gehäuse 40 in Verbindung steht. Die Endkappe 30 weist die Ein- oder Ausströmkammer 10 auf, die an den teilkreisförmigen Kanal 12 angrenzt. Das den Kanal 12 verlassende Fluid wird in die Ein- oder Ausströmkammer 10 geführt und annähernd radialsymmetrisch über die Enden der Hohlfasem eines nicht dargestellten Hohlfaserbündels verteilt.

Der Kanal 12 steht mit dem Zu- oder Ablauf 20 in Verbindung, der in seinem Endbereich die Mündung 320 des Anschlusses 32 der erfindungsgemäßen Filtervorrichtung bildet.

Die Endkappe 30 ist mit der Wulst 36 versehen, die sich radial erstreckt und in deren Endbereich der Anschluß 32 gebildet wird.

In dem in Fig. 1 rechts dargestellten Bereich der Endkappe 30 ist der Anschluß 34 vorgesehen, der die Mündung 340 aufweist, die mit dem zweiten Strömungsraum in Verbindung steht, der durch den die Fasern umgebenden Gehäuseinnenraum 42 gebildet wird.

Die Fig. 2 und 3 zeigen die seitlichen Ansichten der Endkappe 30 gemäß Fig. 1 in den durch die Linien A-A und B-B definierten Perspektiven. Hierbei wird deutlich, daß sowohl der Anschluß 32 als auch der Anschluß 34 radial angeordnet sind. Dies gilt ebenso für die Wulst 36, in deren Endbereich der Anschluß 32 angeordnet ist.

Die Mündungen 320 und 340 der Anschlußstutzen 32 und 34 liegen in zueinander parallelen Ebenen, weisen jedoch wie dies insbesondere aus Fig. 1 hervorgeht in entgegengesetzte Richtungen. Dies kann aus Gründen der Kompatibilität mit bereits vorhandenen Schlauchsystemen bzw. entsprechenden die Filter aufnehmenden Halterungen oder aufgrund von Handhabungsvorteilen erforderlich sein. Grundsätzlich ist es jedoch ebenso möglich, die Anschlüsse auf derselben Seite vorzusehen und die Mündungen in einer Ebene anzuordnen.

Fig. 4 zeigt in einer Draufsicht die Endkappe 30 und verdeutlicht durch die gestrichelte Linie die Anordnung und Ausgestaltung des Kanals 12 sowie der Zu- oder Ablaufleitung 20.

Fig. 4 zeigt, daß die durch den Anschluß 32 verlaufende Zu- oder Ablaufleitung 20 radial angeordnet ist. Die Zulaufleitung 20 geht in den Kanal 12 über, der erfindungsgemäß teilkreisförmig ausgeführt ist und sich in etwa zentrisch über dem Hohlfaserbündel bzw. der Vergußmasse erstreckt.

Der Kanal 12 weist eine in Strömungsrichtung abnehmende Höhe auf, was den Vorteil mit sich bringt, daß das durch den Kanal 12 geführte Fluid pro Winkelabschnitt in gleichen Anteilen, d.h. radialsymmetrisch, den Kanal 12 verläßt und in die Ein- oder Ausströmkammer 10 geführt wird und somit in gleichmäßiger Weise auf die Fasern des Hohlfaserbündels verteilt wird.

Gemäß dem vorliegenden Ausführungsbeispiel ist der Kanal 12 derart ausgeführt, daß dieser in seinem Endbereich die Höhe Null aufweist, d.h. in den Boden der Endkappe 30 übergeht.

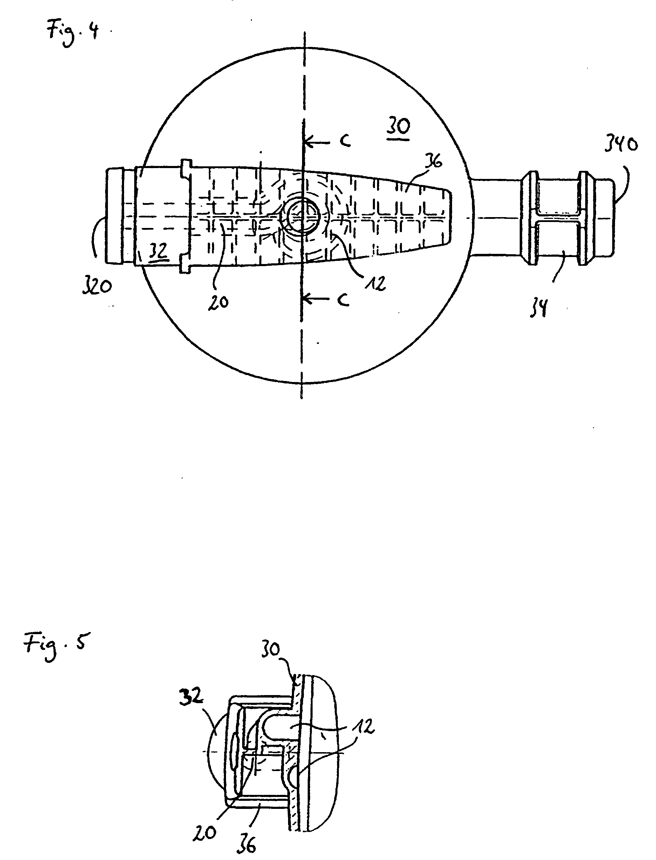

Fig. 5 zeigt in einer Querschnittsdarstellung gemäß Linie C-C nach Fig. 4 die Anordnung und Ausgestaltung des Kanals 12. Hieraus wird deutlich, daß der in Fig. 5, rechts dargestellte Kanalquerschnitt gegenüber dem Zu- oder Ablauf 20 aufgrund der teilkreisförmigen Ausgestaltung versetzt ist und daß die Höhe des Kanals 12 in Strömungsrichtung abnimmt.

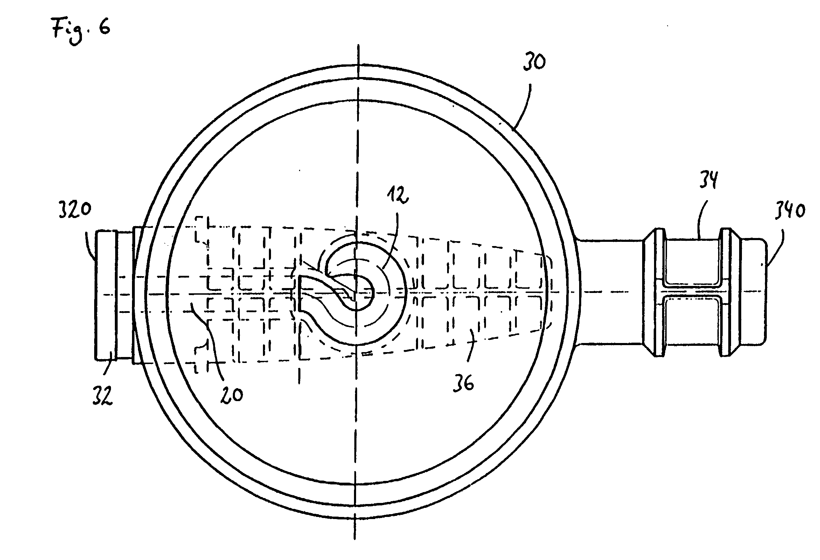

In Fig. 6 ist die Endkappe 30 gemäß Fig. 1 in einer Ansicht von unten dargestellt. Hierbei wird deutlich, daß der Kanal 12 eine teilkreisförmige Gestalt aufweist, und in seinem auslaufenden Endbereich in den Boden der Endkappe 30 übergeht. Fig. 6 zeigt ferner, daß der Mittelpunkt des durch den Kanal 12 gebildeten Teilkreises mit dem Mittelpunkt der Endkappe 30 und somit auch mit dem Mittelpunkt der Vergußmasse bzw. des dem Hohlfaserbündel übereinstimmt

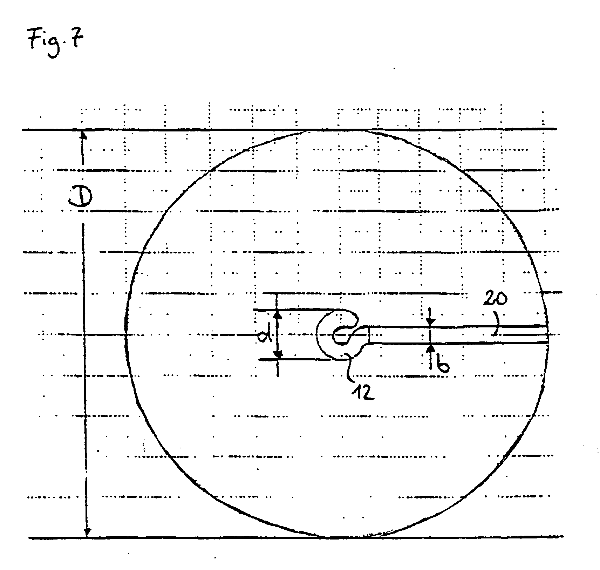

Fig. 7 zeigt eine schematische Darstellung der Größenverhältnisse des Zu- oder Ablaufs 20, des Kanals 12 und des durch den Kreis gekennzeichneten Hohlfaserbündels. Der Durchmesser (d) des kreis- oder teilkreisförmigen Kanals 12 beträgt gemäß dem vorliegenden Ausführungsbeispiel das 2- bis 4-fache, vorzugsweise das 2,5- bis 3,5-fache des Durchmessers (b) des Zu- oder Ablaufes 20. Der Durchmesser (d) des Kanals 12 ist kleiner als 9/16, vorzugsweise kleiner als 3/8 des Durchmessers (D) des Hohlfaserbündels ausgeführt.

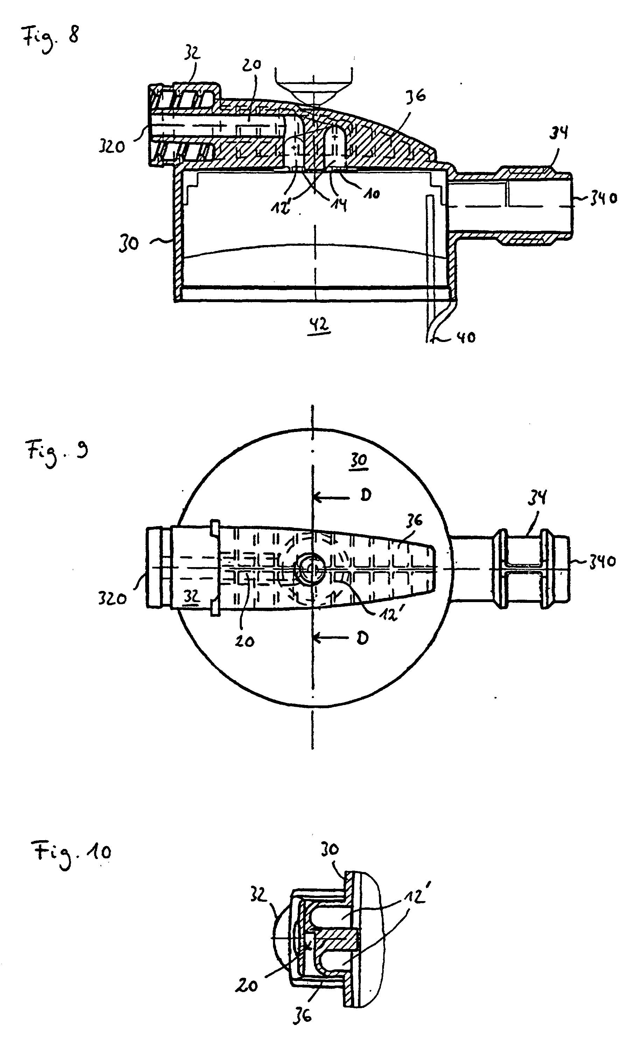

In Fig. 8 ist eine Längsschnittdarstellung einer weiteren Ausführungsform der erfindungsgemäßen Filtervorrichtung dargestellt. Die Endkappe 30 gemäß Fig. 8 weist einen kreisförmigen, d.h. umlaufenden Kanal 12' auf. Die Querschnittsfläche des Kanals 12' nimmt in Strömungsrichtung ab, was dadurch realisiert wird, daß dessen Höhe in Strömungsrichtung verringert wird, wie dies aus Fig. 8 hervorgeht.

In dem sich um den Kanal 12' erstreckenden Bereich sind die gekrümmten Rippen 14 angeordnet, mittels derer das den Kanal 12' verlassende Fluid in definierter Weise gleichmäßig in die Ein- oder Ausströmkammer 10 bzw. über das Hohlfaserbündel verteilt wird. Der Abstand der Rippen 14 zu der nicht näher dargestellten Vergußmasse des Filters ist vorteilhaft derart gering ausgeführt, daß eine fast vollständige Strömungsführung realisiert wird. Der Abstand liegt beispielsweise bei ca. 0,2 mm. Der Abstand des innerhalb des kreisförmigen Kanals 12' liegenden Stutzens der Endkappe 30 zu der Vergußmasse ist vorteilhaft etwas größer, beispielsweise 0,25 mm ausgeführt.

Fig. 9 zeigt eine Draufsicht auf die Endkappe 30 und verdeutlicht, daß der Kanal 12' gemäß dem Ausführungsbeispiel nach Fig. 8 umlaufend ausgeführt ist.

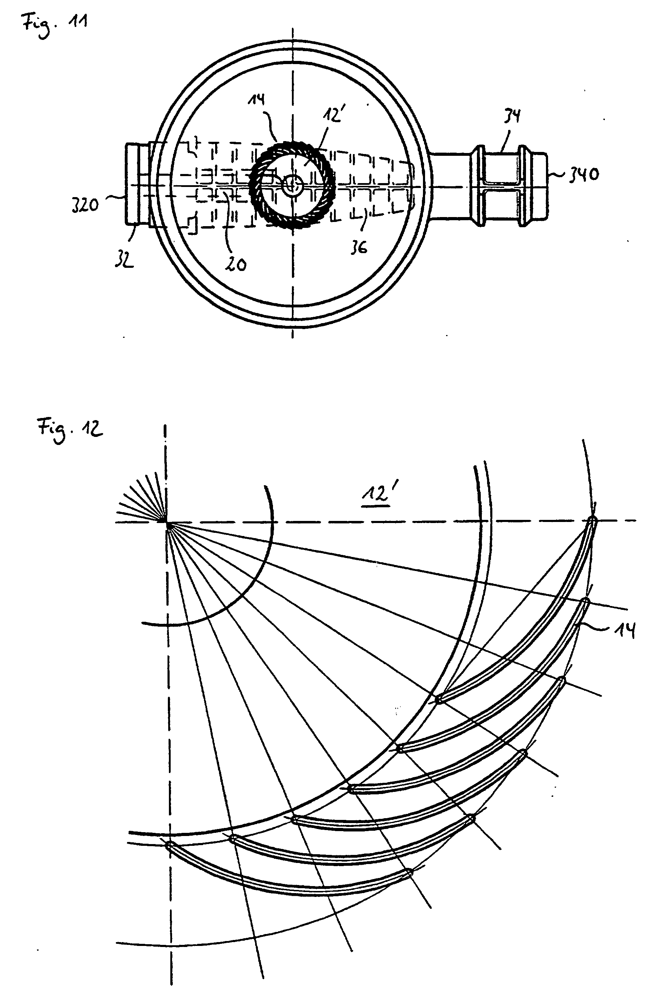

Aus Fig. 10 ist in einer Querschnittsdarstellung gemäß Linie D-D nach Fig. 9 die Anordnung und Ausgestaltung des Kanals 12' ersichtlich. Ein Vergleich mit Fig. 5 zeigt, daß bei der Ausführungsform gemäß Fig. 10 der unten dargestellte Abschnitt des Kanals eine größere Querschnittsfläche aufweist, die auch im weiteren Verlauf entsprechend der umlaufenden Ausführung des Kanals 12' nicht auf 0 zurückgeht.

In Fig. 11 ist die Endkappe gemäß Fig. 8 in einer Ansicht von unten dargestellt. Hierbei wird nochmals deutlich, daß der Kanal 12' umlaufend ausgeführt ist. Ferner erkennt man, daß umlaufend um den Kanal 12' die Rippen 14 angeordnet sind, die nicht geradlinig radial nach außen, sondern gekrümmt angeordnet sind. Selbstverständlich ist neben der gezeigten Ausführung auch jede beliebige andere Anordnung und Ausführung der Rippen 14 denkbar. Die Rippen 14 dienen als Führungselemente für das den Kanal 12' verlassende Fluid. Die Rippen 14 sind beispielsweise ca. 1 mm oder einige 1/10 mm dick. Durch die Ausgestaltung und Anordnung derartiger Führungselemente wird es möglich, das den Kanal 12' verlassende Fluid in definierter Art und Weise und in definierter Richtung in der Ein- oder Ausströmkammer 10 bzw. über das Hohlfaserbündel zu verteilen.

Die Anordnung der Rippen 14 wird aus der Detailansicht gemäß Fig. 12 deutlich. Hierbei wird erkennbar, daß die Rippen 14 dem Kanal 12' unmittelbar benachbart sind. Die Rippen 14 sind äquidistant ausgeführt. Der Abstand der einzelnen Rippen nimmt in Strömungsrichtung ab, was zu einer entsprechenden Beschleunigung des durch die Rippen 14 geführten Fluids führt.

Die Anordnung von Führungselementen, insbesondere von Rippen 14, ist nicht nur bei der umlaufenden Ausführung des Kanals 12' denkbar, sondern selbstverständlich auch bei einer teilkreisförmigen Ausführung gemäß der Fig. 1-7.

Vorteilhafte Dimensionierungen des umlaufenden Kanals 12' gemäß der Fig. 8-12 ergeben sich entsprechend der Erläuterungen zu Fig. 7.

## Patentansprüche

1. Filtervorrichtung zum Stoffaustausch zwischen zwei durch eine als Hohlfaserbündel ausgebildete Membran getrennten Medien, wobei die Faserhohlräume des Hohlfaserbündels einen ersten Strömungsraum bilden und die Filtervorrichtung folgendes umfaßt:
- einen Zu- oder Ablauf (20),
- einen Kanal (12, 12'), der mit dem Zu- oder Ablauf (20) in Verbindung steht, im wesentlichen kreis- oder teilkreisförmig ist, in Richtung zu den Enden der Hohlfasern offen ist, näherungsweise zentrisch zu dem Hohlfaserbündel ist, eine in Strömungsrichtung abnehmende Querschnittsfläche hat und dessen Außendurchmesser kleiner als der Durchmesser des Hohlfaserbündels ist, und
- wenigstens eine Ein- oder Ausströmkammer (10) zur Zu- oder Abfuhr eines Mediums in das oder aus dem Hohlfaserbündel, die mit dem ersten Strömungsraum in Verbindung steht und an den Kanal (12, 12') angrenzt.

2. Filtervorrichtung nach Anspruch 1, **dadurch gekennzeichnet, daß** die Hohlfasern in ihren Endbereichen in einer Vergußmasse aufgenommen sind, die sich parallel zu dem Kanal (12, 12') erstreckt.

3. Filtervorrichtung nach Anspruch 1 oder 2, **dadurch gekennzeichnet, daß** die Höhe und/oder Breite des Kanals (12, 12') in Strömungsrichtung abnimmt.

4. Filtervorrichtung nach Anspruch 3, **dadurch gekennzeichnet, daß** die Querschnittsfläche linear mit dem Strömungsweg abnimmt.

5. Filtervorrichtung nach einem oder mehreren der Ansprüche 1 bis 4, **dadurch gekennzeichnet, daß** sich der Zu- oder Ablauf (20) radial erstreckt, in seinem einen Endbereich in den Kanal (12, 12') übergeht und in seinem anderen Endbereich die Mündung (320) eines Anschlusses (32) der Filtervorrichtung bildet.

6. Filtervorrichtung nach einem oder mehreren der Ansprüche 1 bis 5, **dadurch gekennzeichnet, daß** der Durchmesser des kreis- oder teilkreisförmigen Kanals (12, 12') das 2 bis 4-fache, vorzugsweise das 2,5 bis 3,5-fache des Durchmessers des Zu- oder Ablaufes (20) beträgt.

7. Filtervorrichtung nach einem oder mehreren der Ansprüche 1 bis 6, **dadurch gekennzeichnet, daß** der Durchmesser des kreis- oder teilkreisförmigen Kanals (12, 12') kleiner als 9/16, vorzugsweise kleiner als 3/8 des Durchmessers des Hohlfaserbündels ist.

8. Filtervorrichtung nach einem oder mehreren der Ansprüche 1 bis 7, **dadurch gekennzeichnet, daß** der Kanal (12') kreisförmig ausgeführt ist.

9. Filtervorrichtung nach einem oder mehreren der Ansprüche 1 bis 8, **gekennzeichnet durch** Führungselemente (14) in den dem im wesentlichen kreis- oder teilkreisförmigen Kanal (12, 12') benachbarten Bereichen, mittels derer die Strömungsrichtung des den Kanal (12, 12') verlassenden Fluids beeinflußbar ist.

10. Filtervorrichtung nach Anspruch 9, **dadurch gekennzeichnet, daß** die Führungselemente als gekrümmte Rippen (14) ausgeführt sind.

11. Filtervorrichtung nach Anspruch 10, **dadurch gekennzeichnet, daß** die Rippen (14) im Umfangsrichtung des Kanals (12, 12') äquidistant angeordnet sind.

12. Filtervorrichtung nach einem oder mehreren der Ansprüche 1 bis 11, **gekennzeichnet durch**
- ein rohrabschnittförmiges Gehäuse (40), in das das Hohlfaserbündel aufgenommen ist, wobei ein zweiter Strömungsraum **durch** den das Hohlfaserbündel umgebenden Gehäuseinnenraum (42) gebildet wird, und
- eine mit dem Gehäuse (40) stimseitig in Verbindung stehende Endkappe (30), in der die Ein- oder Ausströmkammer (10) angeordnet ist.

13. Filtervorrichtung nach Anspruch 12, **gekennzeichnet durch** Endkappen (30) in beiden Endbereichen des Gehäuses (40).

14. Filtervorrichtung nach Anspruch 12 oder 13, **dadurch gekennzeichnet, daß** sich das Hohlfaserbündel über die gesamte Querschnittsfläche des Gehäuses (40) erstreckt.

15. Filtervorrichtung nach einem oder mehreren der Ansprüche 12 bis 14, **dadurch gekennzeichnet, daß** jede der Endkappen (30) zwei Anschlüsse (32, 34) aufweist, von denen einer mit dem ersten und einer mit dem zweiten Strömungsraum in Verbindung steht.

16. Filtervorrichtung nach einem oder mehreren der Ansprüche 12 bis 15, **dadurch gekennzeichnet, daß** sich die Anschlüsse (32, 34) radial erstrecken.

17. Filtervorrichtung nach einem oder mehreren der Ansprüche 12 bis 16, **dadurch gekennzeichnet, daß** die Mittellinien der Anschlüsse (32, 34) zueinander parallel verlaufen.

18. Filtervorrichtung nach einem oder mehreren der Ansprüche 12 bis 17, **dadurch gekennzeichnet, daß** die Mündungen (320, 340) der Anschlüsse (32, 34) in einer Ebene oder in zueinander parallelen Ebenen liegen.

19. Filtervorrichtung nach einem oder mehreren der Ansprüche 12 bis 18, **dadurch gekennzeichnet, daß** die Anschlüsse (32, 34) in entgegengesetzte Richtungen weisen.

20. Filtervorrichtung nach einem oder mehreren der Ansprüche 12 bis 19, **dadurch gekennzeichnet, daß** das Gehäuse (40) sowie die Endkappen (30) Spritzgußteile sind.

21. Endkappe für eine Filtervorrichtung gemäß einem oder mehreren der Ansprüche 1 bis 20, wobei die Endkappe (30) folgendes umfaßt:
- einen Zu- oder Ablauf (20),
- einen Kanal (12, 12'), der mit dem Zu- oder Ablauf (20) in Verbindung steht, im wesentlichen kreis- oder teilkreisförmig ist, in Richtung zu den Enden der Hohlfasern eines in einem mit der Endkappe (30) verbindbaren Filtervorrichtungsgehäuse angeordneten Hohlfaserbündels offen und näherungsweise zentrisch dazu ist, eine in Strömungsrichtung abnehmende Querschnittsfläche aufweist und dessen Außendurchmesser kleiner als der Innendurchmesser des das Hohlfaserbündel aufnehmenden Bereiches der Endkappe (30) ist, und
- eine Ein- oder Ausström Kammer, die an den Kanal angrenzt.

## Claims

1. Filter device for exchange of matter between two media separated by a membrane constructed as a hollow fibre bundle, wherein the fibre hollow spaces of the hollow fibre bundle form a first flow chamber and the filter device comprises the following:
- a feed or discharge (20),
- a channel (12, 12'), which is connected to the feed or discharge (20), is substantially circular or in the form of a graduated circle, is open in the direction towards the ends of the hollow fibres, is approximately central to the hollow fibre bundle, has a cross-section area which decreases in the flow direction, and the outer diameter of which is less than the diameter of the hollow fibre bundle, and
- at least one in- or outflow chamber (10) for feeding or removing a medium into or out of the hollow fibre bundle, which is connected to the first flow chamber and adjacent to the channel (12, 12').

2. Filter device according to claim 1, **characterized in that** the hollow fibres are accommodated in their end regions in a sealing compound which extends parallel to the channel (12, 12').

3. Filter device according to claim 1 or 2, **characterized in that** the height and/or width of the channel (12, 12') decreases in the flow direction.

4. Filter device according to claim 3, **characterized in that** the cross-section area decreases linearly with the flow path.

5. Filter device according to one or more of claims 1 to 4, **characterized in that** the feed or discharge (20) extends radially, merges in its one end region into the channel (12, 12') and in its other end region forms the opening (320) of a connection (32) of the filter device.

6. Filter device according to one or more of claims 1 to 5, **characterized in that** the diameter of the channel (12, 12') which is circular or in the form of a graduated circle is 2 to 4 times, preferably 2.5 to 3.5 times the diameter of the feed or discharge (20).

7. Filter device according to one or more of claims 1 to 6, **characterized in that** the diameter of the channel (12, 12') which is circular or in the form of a graduated circle is less than 9/16, preferably less than 3/8 of the diameter of the hollow fibre bundle.

8. Filter device according to one or more of claims 1 to 7, **characterized in that** the channel (12') is circular in construction.

9. Filter device according to one or more of claims 1 to 8, **characterized by** guide elements (14) in the regions adjacent to the channel (12, 12') which is substantially circular or in the form of a graduated circle, by means of which the flow direction of the fluid leaving the channel (12, 12') can be influenced.

10. Filter device according to claim 9, **characterized in that** the guide elements are constructed as curved ribs (14).

11. Filter device according to claim 10, **characterized in that** the ribs (14) are arranged equidistantly in the circumferential direction of the channel (12, 12').

12. Filter device according to one or more of claims 1 to 11, **characterized by**
- a housing (40) in the form of a tubular section, in which the hollow fibre bundle is accommodated, wherein a second flow chamber is formed by the housing inner space (42) surrounding the hollow fibre bundle, and
- an end cap (30) which is connected to the housing (40) on the face and in which the in- or outflow chamber (10) is arranged.

13. Filter device according to claim 12, **characterized by** end caps (30) in both end regions of the housing (40).

14. Filter device according to claim 12 or 13, **characterized in that** the hollow fibre bundle extends over the entire cross-section area of the housing (40).

15. Filter device according to one or more of claims 12 to 14, **characterized in that** each of the end caps (30) has two connections (32, 34), of which one is connected to the first and one to the second flow chamber.

16. Filter device according to one or more of claims 12 to 15, **characterized in that** the connections (32, 34) extend radially.

17. Filter device according to one or more of claims 12 to 16, **characterized in that** the central lines of the connections (32, 34) run parallel to one another.

18. Filter device according to one or more of claims 12 to 17, **characterized in that** the openings (320, 340) of the connections (32, 34) lie in one plane or in planes which are parallel to one another.

19. Filter device according to one or more of claims 12 to 18, **characterized in that** the connections (32, 34) point in opposite directions.

20. Filter device according to one or more of claims 12 to 19, **characterized in that** the housing (40) and the end caps (30) are injection-moulded components.

21. End cap for a filter device according to one or more of claims 1 to 20, wherein the end cap (30) comprises the following:
- a feed or discharge (20),
- a channel (12, 12'), which is connected to the feed or discharge (20), is substantially circular or in the form of a graduated circle, is open in the direction towards the ends of the hollow fibres of a hollow fibre bundle arranged in a filter device housing which can be connected to the end cap (30) and is arranged approximately centrally thereto, has a cross-section area which decreases in the flow direction, and the outer diameter of which is less than the internal diameter of the region of the end cap (30) which accommodates the hollow fibre bundle, and
- an in- or outflow chamber which is adjacent to the channel.

## Revendications

1. Dispositif de filtration pour l'échange de matière entre deux milieux séparés par une membrane réalisée comme paquet de fibres creuses, où les espaces creux des fibres du paquet de fibres creuses forment une première enceinte d'écoulement, et le dispositif de filtration comprend :
- un conduit d'admission ou d'écoulement (20),
- un canal (12, 12') qui est en liaison avec le conduit d'admission ou d'écoulement (20), qui est sensiblement en forme de cercle ou en forme de cercle partiel, qui est ouvert en direction des extrémités des fibres creuses, qui est approximativement centrique au paquet de fibres creuses, qui présente une section transversale diminuant dans la direction d'écoulement et dont le diamètre extérieur est plus petit que le diamètre du paquet de fibres creuses ; et
- au moins une enceinte d'admission ou d'écoulement (10) pour l'admission ou l'évacuation d'un milieu dans le paquet de fibres creuses ou hors de celui-ci, qui est en liaison avec la première enceinte d'écoulement et qui avoisine le canal (12, 12').

2. Dispositif de filtration selon la revendication 1, **caractérisé en ce que** les fibres creuses sont reçues dans leurs zones d'extrémité dans une masse de scellement qui s'étend parallèlement au canal (12, 12').

3. Dispositif de filtration selon la revendication 1 ou 2, **caractérisé en ce que** la hauteur et/ou largeur du canal (12, 12') diminue dans la direction d'écoulement.

4. Dispositif de filtration selon la revendication 3, **caractérisé en ce que** la face en section transversale diminue linéairement avec le chemin d'écoulement.

5. Dispositif de filtration selon l'une ou plusieurs des revendications 1 à 4, **caractérisé en ce que** le conduit d'admission ou d'écoulement (20) s'étend radialement, rejoint par une zone d'extrémité le canal (12, 12') et forme à son autre zone d'extrémité l'embouchure (320) d'un raccordement (32) du dispositif de filtration.

6. Dispositif de filtration selon l'une ou plusieurs des revendications 1 à 5, **caractérisé en ce que** le diamètre du canal (12, 12') en forme de cercle ou en forme de cercle partiel représente 2 à 4 fois, de préférence 2,5 à 3,5 fois le diamètre du conduit d'admission ou d'écoulement (20).

7. Dispositif de filtration selon l'une ou plusieurs des revendications 1 à 6, **caractérisé en ce que** le diamètre du canal (12, 12') en forme de cercle ou en forme de cercle partiel est plus petit que 9/16, de préférence plus petit que 3/8 du diamètre du paquet de fibres creuses.

8. Dispositif de filtration selon l'une ou plusieurs des revendications 1 à 7, **caractérisé en ce que** le canal (12') est réalisé en forme de cercle.

9. Dispositif de filtration selon l'une ou plusieurs des revendications 1 à 8, **caractérisé par** des éléments de guidage (14) dans les zones avoisinant le canal (12, 12') sensiblement en forme de cercle ou de cercle partiel, au moyen desquels on peut agir sur la direction d'écoulement du fluide quittant le canal (12, 12').

10. Dispositif de filtration selon la revendication 9, **caractérisé en ce que** les éléments de guidage sont réalisés comme des nervures courbées (14).

11. Dispositif de filtration selon la revendication 10, **caractérisé en ce que** les nervures (14) sont disposées d'une manière équidistante dans la direction périphérique du canal (12, 12').

12. Dispositif de filtration selon l'une ou plusieurs des revendications 1 à 11, **caractérisé par** :
- un boîtier (40) en forme de tronçon tubulaire, dans lequel est reçu le paquet de fibres creuses, où une deuxième enceinte d'écoulement est formée par l'espace intérieur (42) du boîtier entourant le paquet de fibres creuses ; et
- un capuchon d'extrémité (30) en liaison, au côté frontal, avec le boîtier (40), dans lequel est disposée la chambre d'admission ou d'écoulement (10).

13. Dispositif de filtration selon la revendication 12, **caractérisé par** des capuchons d'extrémité (30) dans les deux zones d'extrémité du boîtier (40).

14. Dispositif de filtration selon la revendication 12 ou 13, **caractérisé en ce que** le paquet de fibres creuses s'étend sur toute la face en section transversale du boîtier (40).

15. Dispositif de filtration selon l'une ou plusieurs des revendications 12 à 14, **caractérisé en ce que** chacun des capuchons d'extrémité (30) présente deux raccords (32, 34) dont un est en liaison avec la première enceinte d'écoulement et un avec la seconde enceinte d'écoulement.

16. Dispositif de filtration selon l'une ou plusieurs des revendications 12 à 15, **caractérisé en ce que** les raccords (32, 34) s'étendent radialement.

17. Dispositif de filtration selon l'une ou plusieurs des revendications 12 à 16, **caractérisé en ce que** les lignes médianes des raccords (32, 34) s'étendent parallèlement l'une à l'autre.

18. Dispositif de filtration selon l'une ou plusieurs des revendications 12 à 17, **caractérisé en ce que** les embouchures (320, 340) des raccords (32, 34) se situent dans un plan ou dans des plans parallèles l'un à l'autre.

19. Dispositif de filtration selon l'une ou plusieurs des revendications 12 à 18, **caractérisé en ce que** les raccords (32, 34) sont dirigés dans des directions opposées.

20. Dispositif de filtration selon l'une ou plusieurs des revendications 12 à 19, **caractérisé en ce que** le boîtier (40) ainsi que les capuchons d'extrémité (30) sont des pièces moulées par injection.

21. Capuchon d'extrémité pour un dispositif de filtration selon l'une ou plusieurs des revendications 1 à 20, où le capuchon d'extrémité (30) comprend :
- un conduit d'admission ou d'écoulement (20),
- un canal (12, 12') qui est en liaison avec le conduit d'admission ou d'écoulement (20) qui est sensiblement en forme de cercle ou en forme de cercle partiel, qui est ouvert en direction des extrémités des fibres creuses d'un paquet de fibres creuses disposé dans un boîtier du dispositif de filtration pouvant être relié au capuchon d'extrémité (30) et qui est approximativement centrique à celui-ci, qui présente une face en section transversale diminuant dans la direction d'écoulement, et dont le diamètre extérieur est plus petit que le diamètre intérieur de la zone du capuchon d'extrémité (30) recevant le paquet de fibres creuses, et
- une chambre d'admission ou d'écoulement qui avoisine le canal.
